(19) Europäisches Patentamt
European Patent Office
Office européen des brevets

(11) Veröffentlichungsnummer: **0 420 020 A2**

(12) # EUROPÄISCHE PATENTANMELDUNG

(21) Anmeldenummer: **90118007.5**

(22) Anmeldetag: **19.09.90**

(51) Int. Cl.5: **C07D 233/60**, C07D 249/08, A01N 43/50, A01N 43/653

(30) Priorität: **28.09.89 DE 3932387**

(43) Veröffentlichungstag der Anmeldung: **03.04.91 Patentblatt 91/14**

(84) Benannte Vertragsstaaten: **AT BE CH DE DK ES FR GB GR IT LI NL SE**

(71) Anmelder: **BASF Aktiengesellschaft**
**Carl-Bosch-Strasse 38**
**W-6700 Ludwigshafen(DE)**

(72) Erfinder: **Seele, Rainer, Dr.**
**Leiblstrasse 3**
**W-6701 Fussgoenheim(DE)**
Erfinder: **Rohr, Wolfgang, Dr.**
**In der Dreispitz 13**
**W-6706 Wachenheim(DE)**
Erfinder: **Zipperer, Bernhard, Dr.**
**Am Herrgottsacker 6**
**W-6716 Dirmstein(DE)**
Erfinder: **Karbach, Stefan, Dr.**
**Grundwiesenweg 44**
**W-6730 Neustadt(DE)**
Erfinder: **Rueb, Lothar, Dr.**
**Am Schoeneck 11**
**W-6720 Speyer(DE)**
Erfinder: **Ammermann, Eberhard, Dr.**
**Sachsenstrasse 3**
**W-6700 Ludwigshafen(DE)**
Erfinder: **Lorenz, Gisela, Dr.**
**Erlenweg 13**
**W-6730 Neustadt(DE)**
Erfinder: **Rademacher, Wilhelm, Dr.**
**Austrasse 1**
**W-6703 Limburgerhof(DE)**
Erfinder: **Jung, Johann, Prof. Dr.**
**Hardenburgstrasse 19**
**W-6703 Limburgerhof(DE)**

(54) **Vinylazole und ihre Verwendung als Pflanzenschutzmittel.**

(57) Vinylazole der allgemeinen Formel I

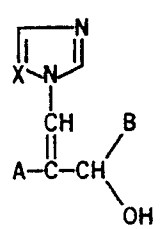

I

in welcher
A und B
Alkyl, Cycloalkyl, Tetrahydropyranyl, Pyridyl, Naphthyl, Biphenyl oder Phenyl bedeuten, wobei diese Reste substituiert sein können,
X
den Rest CH oder N bedeutet,
sowie deren für Pflanzen verträgliche Säureadditionssalze und Metallkomplexe und diese Verbindungen enthaltende Pflanzenschutzmittel.

EP 0 420 020 A2

# VINYLAZOLE UND IHRE VERWENDUNG ALS PFLANZENSCHUTZMITTEL

Die vorliegende Erfindung betrifft neue Azolverbindungen, Verfahren zu ihrer Herstellung und diese enthaltende Fungizide und Wachstumsregulatoren.

Es ist bekannt, 1-(1,2,4-Triazol-1-yl)-2-(4-chlorphenyl)-3-(2,4-dichlorphenyl)-1-propen-3-ol und 1-(1,2,4-Triazol-1-yl)-2-(4-chlorphenyl)-3-phenyl-1-propen-3-ol (EP 23286) als Fungizide zu verwenden. Die fungiziden Wirkungen sind jedoch nicht in allen Fällen befriedigend.

Es wurde nun gefunden, daß Vinylazole der allgemeinen Formel I,

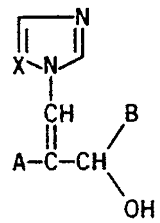

I

in welcher

A und B

gleich oder verschieden sind und $C_1$-$C_8$-Alkyl, $C_3$-$C_8$-Cycloalkyl, Tetrahydropyranyl, Pyridyl, Naphthyl, Biphenyl oder Phenyl bedeuten, wobei diese Reste einfach bis dreifach durch Halogen, Nitro, Phenoxy, Amino, Alkyl, Alkoxy oder Halogenalkyl mit jeweils 1 bis 4 C-Atomen substituiert sein können,

X

den Rest CH oder N bedeutet,

sowie deren für Pflanzen verträgliche Säureadditionssalze oder Metallkomplexe außer den Verbindungen, in denen gleichzeitig A gegebenenfalls durch Chlor substituiertes Phenyl und B gegebenenfalls durch Chlor in 4-Stellung oder 2,4-Stellung substituiertes Phenyl bedeutet, eine bessere fungizide und wachstumsregulatorische Wirkung besitzen als bekannte Azolverbindungen.

Die Verbindungen der Formel I enthalten asymmetrische C-Atome und können daher als Enantiomere und Diastereomere auftreten. Die Gemische von Diastereomeren lassen sich bei den erfindungsgemäßen Verbindungen in übli cher Weise, beispielsweise aufgrund ihrer unterschiedlichen Löslichkeit oder durch Säulenchromatographie trennen und in reiner Form isolieren. Die Racemate lassen sich bei den erfindungsgemäßen Verbindungen nach bekannten Methoden, beispielsweise durch Salzbildung mit einer optisch aktiven Säure, Trennung der diastereomeren Salze und Freisetzung der Enantiomeren mittels einer Base trennen. Als fungizide und wachstumsregulatorische Mittel kann man die einheitlichen Diastereomere bzw. Enantiomere als auch deren bei der Synthese anfallenden Gemische verwenden. Alle diese Verbindungen werden von der Erfindung umfaßt.

A bzw. B bedeuten beispielsweise $C_1$-$C_8$-Alkyl, insbesondere $C_1$-$C_4$-Alkyl, wie Methyl, Ethyl, Isopropyl, n-Propyl, n-Butyl, sec-Butyl, tert.-Butyl, n-Pentyl, Neopentyl, 1-Naphthyl, 2-Naphthyl, p-Biphenyl, Phenyl, Halogenphenyl, wie 2-Chlorphenyl, 2-Fluorphenyl, 2-Bromphenyl, 3-Chlorphenyl, 3-Bromphenyl, 3-Fluorphenyl, 4-Fluorphenyl, 4-Chlorphenyl, 4-Bromphenyl, 2,4-Dichlorphenyl, 2,3-Dichlorphenyl, 2,5-Dichlorphenyl, 2,6-Dichlorphenyl, 2-Chlor-6-fluorphenyl, 2-Chlor-4-fluorphenyl, $C_1$-$C_4$-Alkoxyphenyl, wie 2-Methoxyphenyl, 3-Methoxyphenyl, 4-Methoxyphenyl, 2,4-Dimethoxyphenyl, $C_1$-$C_4$-Alkylphenyl, wie 4-Ethylphenyl, 4-Isopropylphenyl, 4-tert.-Butylphenyl, 4-tert.-Butyloxyphenyl, 2-Chlor-6-methylphenyl, 3,4-Dimethoxyphenyl, 3-Phenoxyphenyl, 4-Phenoxyphenyl, 3-Nitrophenyl, 4-Nitrophenyl, 3-Aminophenyl, 4-Aminophenyl, 2-Trifluormethylphenyl, 3-Trifluormethylphenyl, 4-Trifluormethylphenyl, 3-Pyridyl, Tetrahydropyranyl, $C_3$-$C_6$-Cycloalkyl, wie Cyclopropyl, Cyclopentyl, Cyclohexyl.

Es bedeuten nicht gleichzeitig A Phenyl, Monochlorphenyl, Dichlorphenyl und B Phenyl, 4-Chlorphenyl, 2,4-Dichlorphenyl.

Bei der Bedeutung B wird in 2-Stellung substituiertes Phenyl bevorzugt.

Säureadditionssalze sind beispielsweise die Hydrochloride, Bromide, Sulfate, Nitrate, Phosphate, Oxalate oder Dodecylbenzolsulfonate. Die Wirksamkeit der Salze geht auf das Kation zurück, so daß es auf das Anion im allgemeinen nicht ankommt. Die erfindungsgemäßen Wirkstoffsalze werden hergestellt durch Umsetzung der Vinylazole (I) mit geeigneten Säuren.

Metallkomplexe der Wirkstoffe I oder ihrer Salze können z.B. mit Kupfer, Zink, Zinn, Mangan, Eisen, Kobalt oder Nickel gebildet werden, indem man die Vinylazolderivate mit den entsprechenden Metallsalzen

umsetzt.

Die Verbindungen der Formel I können hergestellt werden, indem man eine Verbindung der Formel II,

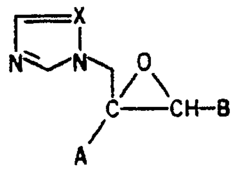

II

in welcher A, B und X die oben angegebene Bedeutung haben, in Gegenwart einer Base in die Vinylazole umlagert.

Die Reaktion erfolgt gegebenenfalls in Gegenwart eines Lösungs-oder Verdünnungsmittels unter Zusatz einer anorganischen oder organischen Base bei Temperaturen zwischen 10 und 120 °C.

Zu den bevorzugten Lösungs- und Verdünnungsmitteln gehören Ketone, wie Aceton, Methylethylketon oder Cyclohexanon, Nitrile wie Acetonitril oder Propionitril, Alkohole wie Methanol, Ethanol, iso-Propanol, n-Butanol oder Glycol, Ether wie Tetrahydrofuran, Diethylether, Dimethoxyethan, Dioxan oder Diisopropylether, Amide wie Dimethylformamid, Dimethylacetamid oder N-Methylpyrrolidon, Sulfolan oder entsprechende Gemische.

Geeignete Basen sind beispielsweise Alkalihydroxide wie Lithium-, Natrium-oder Kaliumhydroxid, Alkalihydride wie Lithium-, Natrium- und Kaliumhydrid, Alkaliamide wie Natrium- und Kaliumamid, Alkalicarbonate wie Natrium- oder Kaliumcarbonat oder Natrium-, Kaliumhydrogencarbonat, ferner Natrium- oder Kalium-tert.-butoxid, sowie Natrium- oder Kaliummethanolat.

Die Umsetzung wird im allgemeinen bei Temperaturen zwischen 20 und 150 °C durchgeführt.

Die Ausgangsverbindungen II können nach bekannten Methoden (siehe EP 94564 und EP 196038) hergestellt werden.

Beispiele

Das nachfolgende Beispiel erläutert die Herstellung der Wirkstoffe.

Beispiel 1

1-(1,2,4-Triazol-1-yl)-2-(4-chlorphenyl)-3-(2-trifluormethyl-phenyl)-1-propen-3-ol

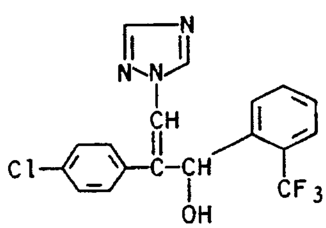

4 g 2-(1,2,4-Triazol-1-ylmethyl)-2-(4-chlorphenyl)-3-(2-trifluor-methylphenyl) -oxiran werden in 80 ml Methanol gelöst und mit 1,2 g Natriummethylat versetzt und für eine Stunde unter Rückfluß erhitzt. Anschließend wird die Lösung bei Raumtemperatur (20 °C) mit 50 ml Wasser versetzt und mehrmals mit Methyl-tert.-butylether ausgeschüttelt. Die isolierte organische Phase wird zweimal mit Wasser gewaschen, daraufhin über Natriumsulfat getrocknet und eingeengt, wobei 3,8 g (95 %) 1-(1,2,4-Triazol-1-yl)-2-(4-chlorphenyl)-3-(2-trifluormethylphenyl)-1-propen-3-ol erhalten werden, Fp.: 161 °C.

Entsprechend Beispiel 1 können die in der Tabelle aufgeführten Verbindungen hergestellt werden.

Tabelle:

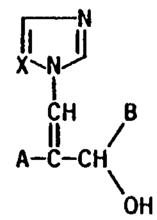

I

| Beispiel | A | B | X | Schmp/IR |
|---|---|---|---|---|
| 1 | 4-Cl-$C_6H_4$ | 2-$CF_3$-$C_6H_4$ | N | 161°C |
| 2 | 4-Cl-$C_6H_4$ | 2-Cl-$C_6H_4$ | N | |
| 3 | 4-Cl-$C_6H_4$ | 2-F-$C_6H_4$ | N | |
| 4 | 4-Cl-$C_6H_4$ | 2-Br-$C_6H_4$ | N | |
| 5 | 4-Cl-$C_6H_4$ | 2-$CH_3$-$C_6H_4$ | N | 116-169°C |
| 6 | 4-Cl-$C_6H_4$ | 2-$OCH_3$-$C_6H_4$ | N | 1491, 1245, 1091, 1015, 756 $cm^{-1}$ |
| 7 | 4-Cl-$C_6H_4$ | Cyclopropyl | N | |
| 8 | 4-Cl-$C_6H_4$ | Cyclopentyl | N | |
| 9 | 4-Cl-$C_6H_4$ | Cyclohexyl | N | |
| 10 | 4-Cl-$C_6H_4$ | 4-Tetrahydropyranyl | N | |
| 11 | 4-Cl-$C_6H_4$ | 3-Pyridyl | N | |
| 12 | 4-Cl-$C_6H_4$ | $CH_3$ | N | |
| 13 | $C_6H_5$ | 2-$CF_3$-$C_6H_4$ | N | 172-174°C |
| 14 | $C_6H_5$ | 2-Cl-$C_6H_4$ | N | |
| 15 | $C_6H_5$ | 2-F-$C_6H_4$ | N | |
| 16 | $C_6H_5$ | 2-Br-$C_6H_4$ | N | |
| 17 | $C_6H_5$ | 2-$CH_3$-$C_6H_4$ | N | |
| 18 | $C_6H_5$ | 2-$OCH_3$-$C_6H_4$ | N | |

Tabelle (Fortsetzung)

| Beispiel | A | B | X | Schmp/IR |
|---|---|---|---|---|
| 19 | $C_6H_5$ | Cyclopropyl | N | |
| 20 | $C_6H_5$ | Cyclopentyl | N | |
| 21 | $C_6H_5$ | Cyclohexyl | N | |
| 22 | $C_6H_5$ | 4-Tetrahydropyranyl | N | |
| 23 | $C_6H_5$ | 3-Pyridyl | N | |
| 24 | $4-F-C_6H_4$ | $2-CF_3-C_6H_4$ | N | |
| 25 | $4-F-C_6H_4$ | $2-Cl-C_6H_4$ | N | 159-160°C |
| 26 | $4-F-C_6H_4$ | $2-Cl-C_6H_4$ | N x $CuCl_2$ | 181-185°C |
| 27 | $4-F-C_6H_4$ | $2-Cl-C_6H_4$ | CH | 175-177°C |
| 28 | $4-F-C_6H_4$ | $2-F-C_6H_4$ | N | |
| 29 | $4-F-C_6H_4$ | $2-Br-C_6H_4$ | N | 176-178°C |
| 30 | $4-F-C_6H_4$ | $2-CH_3-C_6H_4$ | N | 116-168°C |
| 31 | $4-F-C_6H_4$ | $2-OCH_3-C_6H_4$ | N | 78-80°C |
| 32 | $4-F-C_6H_4$ | Cyclopropyl | N | |
| 33 | $4-F-C_6H_4$ | Cyclopentyl | N | |
| 34 | $4-F-C_6H_4$ | Cyclohexyl | N | |
| 35 | $4-F-C_6H_4$ | 3-Pyridyl | N | |
| 36 | $4-F-C_6H_4$ | 4-Tetrahydropyranyl | N | |
| 37 | $4-Br-C_6H_4$ | $2-CF_3-C_6H_4$ | N | |
| 38 | $4-Br-C_6H_4$ | $2-F-C_6H_4$ | N | |
| 39 | $4-Br-C_6H_4$ | $2-Cl-C_6H_4$ | N | |
| 40 | $4-Br-C_6H_4$ | $2-Br-C_6H_4$ | N | |
| 41 | $4-Br-C_6H_4$ | $2-CH_3-C_6H_4$ | N | |
| 42 | $4-Br-C_6H_4$ | $2-OCH_3-C_6H_4$ | N | |

Tabelle (Fortsetzung)

| Beispiel | A | B | X | Schmp/IR |
|---|---|---|---|---|
| 43 | 4-Br-$C_6H_4$ | Cyclopropyl | N | |
| 44 | 4-Br-$C_6H_4$ | Cyclohexyl | N | |
| 45 | 2-F-$C_6H_4$ | 2-$CF_3$-$C_6H_4$ | N | |
| 46 | 2-F-$C_6H_4$ | 2-F-$C_6H_4$ | N | 104-106°C |
| 47 | 2-F-$C_6H_4$ | 2-Cl-$C_6H_4$ | N | |
| 48 | 2-F-$C_6H_4$ | 2-Br-$C_6H_4$ | N | |
| 49 | 2-F-$C_6H_4$ | 2-$CH_3$-$C_6H_4$ | N | |
| 50 | 2-F-$C_6H_4$ | 2-$OCH_3$-$C_6H_4$ | N | |
| 51 | 2-F-$C_6H_4$ | Cyclopentyl | N | |
| 52 | 2-F-$C_6H_4$ | Cyclohexyl | N | |
| 53 | 2-Cl-$C_6H_4$ | 2-$CF_3$-$C_6H_4$ | N | |
| 54 | 2-Cl-$C_6H_4$ | 2-F-$C_6H_4$ | N | |
| 55 | 2-Cl-$C_6H_4$ | 2-Cl-$C_6H_4$ | N | |
| 56 | 2-Cl-$C_6H_4$ | 2-Br-$C_6H_4$ | N | |
| 57 | 2-Cl-$C_6H_4$ | 2-$CH_3$-$C_6H_4$ | N | |
| 58 | 2-Cl-$C_6H_4$ | 2-$OCH_3$-$C_6H_4$ | N | |
| 59 | 2-Cl-$C_6H_4$ | Cyclopentyl | N | |
| 60 | 2-Cl-$C_6H_4$ | Cyclohexyl | N | |
| 61 | 2-Br-$C_6H_4$ | 2-$CF_3$-$C_6H_4$ | N | |
| 62 | 2-Br-$C_6H_4$ | 2-F-$C_6H_4$ | N | |
| 63 | 2-Br-$C_6H_4$ | 2-Cl-$C_6H_4$ | N | |
| 64 | 2-Br-$C_6H_4$ | 2-Br-$C_6H_4$ | N | |
| 65 | 2-Br-$C_6H_4$ | 2-$CH_3$-$C_6H_4$ | N | |
| 66 | 2-Br-$C_6H_4$ | 2-$OCH_3$-$C_6H_4$ | N | |

Tabelle (Fortsetzung)

| Beispiel | A | B | X | Schmp/IR |
|---|---|---|---|---|
| 67 | 2-Br-$C_6H_4$ | Cyclohexyl | N | |
| 68 | 3-Cl-$C_6H_4$ | 2-$CF_3$-$C_6H_4$ | N | |
| 69 | 3-Cl-$C_6H_4$ | 2-F-$C_6H_4$ | N | |
| 70 | 3-Cl-$C_6H_4$ | 2-Cl-$C_6H_4$ | N | |
| 71 | 3-Cl-$C_6H_4$ | 2-Br-$C_6H_4$ | N | |
| 72 | 3-Cl-$C_6H_4$ | 2-$CH_3$-$C_6H_4$ | N | |
| 73 | 3-Cl-$C_6H_4$ | Cyclohexyl | N | |
| 74 | 2,4-$Cl_2$-$C_6H_3$ | 2-$CF_3$-$C_6H_4$ | N | |
| 75 | 2,4-$Cl_2$-$C_6H_3$ | 2-F-$C_6H_4$ | N | |
| 76 | 2,4-$Cl_2$-$C_6H_3$ | 2-Cl-$C_6H_4$ | N | |
| 77 | 2,4-$Cl_2$-$C_6H_3$ | 2-Br-$C_6H_4$ | N | |
| 78 | 2,4-$Cl_2$-$C_6H_3$ | 2-$CH_3$-$C_6H_4$ | N | |
| 79 | 2,4-$Cl_2$-$C_6H_3$ | 2-$OCH_3$-$C_6H_4$ | N | |
| 80 | 2,4-$Cl_2$-$C_6H_3$ | 3-Pyridyl | N | |
| 81 | 2,4-$Cl_2$-$C_6H_3$ | Cyclopropyl | N | |
| 82 | 2,4-$Cl_2$-$C_6H_3$ | Cyclopentyl | N | |
| 83 | 2,4-$Cl_2$-$C_6H_3$ | Cyclohexyl | N | |
| 84 | 2,4-$Cl_2$-$C_6H_3$ | 4-Tetrahydropyranyl | N | |
| 85 | 2-Cl-4-F-$C_6H_3$ | 2-$CF_3$-$C_6H_4$ | N | |
| 86 | 2-Cl-4-F-$C_6H_3$ | 2-$CF_3$-$C_6H_4$ | CH | |
| 87 | 2-Cl-4-F-$C_6H_3$ | 2-F-$C_6H_4$ | N | |
| 88 | 2-Cl-4-F-$C_6H_3$ | 2-Cl-$C_6H_4$ | N | |
| 89 | 2-Cl-4-F-$C_6H_3$ | 2-$CH_3$-$C_6H_4$ | N | |
| 90 | 2-$CF_3$-$C_6H_4$ | 2-$CF_3$-$C_6H_4$ | N | |

Tabelle (Fortsetzung)

| Beispiel | A | B | X | Schmp/IR |
|---|---|---|---|---|
| 91 | $2-CF_3-C_6H_4$ | $2-F-C_6H_4$ | N | |
| 92 | $2-CF_3-C_6H_4$ | $2-Cl-C_6H_4$ | N | |
| 93 | $2-CF_3-C_6H_4$ | $2-Br-C_6H_4$ | N | |
| 94 | $2-CF_3-C_6H_4$ | $2-CH_3-C_6H_4$ | N | |
| 95 | $2-CF_3-C_6H_4$ | $2-OCH_3-C_6H_4$ | N | |
| 96 | $2-CF_3-C_6H_4$ | 3-Pyridyl | N | |
| 97 | $2-CF_3-C_6H_4$ | Cyclopropyl | N | |
| 98 | $2-CF_3-C_6H_4$ | Cyclopentyl | N | |
| 99 | $2-CF_3-C_6H_4$ | Cyclohexyl | N | |
| 100 | $2-CF_3-C_6H_4$ | 4-Tetrahydropyranol | N | |
| 101 | $3-CF_3-C_6H_4$ | $2-CF_3-C_6H_4$ | N | |
| 102 | $3-CF_3-C_6H_4$ | $2-F-C_6H_4$ | N | |
| 103 | $3-CF_3-C_6H_4$ | $2-Cl-C_6H_4$ | N | |
| 104 | $3-CF_3-C_6H_4$ | $2-Br-C_6H_4$ | N | |
| 105 | $3-CF_3-C_6H_4$ | $2-CH_3-C_6H_4$ | N | |
| 106 | $3-CF_3-C_6H_4$ | $2-OCH_3-C_6H_4$ | N | |
| 107 | $4-CF_3-C_6H_4$ | $2-CF_3-C_6H_4$ | N | |
| 108 | $4-CF_3-C_6H_4$ | $2-F-C_6H_4$ | N | |
| 109 | $4-CF_3-C_6H_4$ | $2-Cl-C_6H_4$ | N | |
| 110 | $4-CF_3-C_6H_4$ | $2-Br-C_6H_4$ | N | |
| 111 | $4-CF_3-C_6H_4$ | $2-CH_3-C_6H_4$ | N | |
| 112 | $4-CF_3-C_6H_4$ | $2-OCH_3-C_6H_4$ | N | |
| 113 | $4-CF_3-C_6H_4$ | Cyclopentyl | N | |
| 114 | $4-CF_3-C_6H_4$ | Cyclohexyl | N | |

Tabelle (Fortsetzung)

| Beispiel | A | B | X | Schmp/IR |
|---|---|---|---|---|
| 115 | 1-Naphthyl | 2-Cl-$C_6H_4$ | N | |
| 116 | 1-Naphthyl | 2-Br-$C_6H_4$ | N | |
| 117 | 1-Naphthyl | 2-F-$C_6H_4$ | N | |
| 118 | 2-Naphthyl | 2-F-$C_6H_4$ | N | |
| 119 | 2-Naphthyl | 2-Cl-$C_6H_4$ | N | |
| 120 | 2-Naphthyl | 2-Br-$C_6H_4$ | N | |
| 121 | 2-Naphthyl | 2-$CH_3$-$C_6H_4$ | N | |
| 122 | 2-Naphthyl | 2-$OCH_3$-$C_6H_4$ | N | |
| 123 | 2-Naphthyl | Cyclohexyl | N | |
| 124 | 4-Biphenyl | 2-F-$C_6H_4$ | N | |
| 125 | 4-Biphenyl | 2-Cl-$C_6H_4$ | N | |
| 126 | 4-Biphenyl | 2-Br-$C_6H_4$ | N | |
| 127 | 4-$CH_3$-$C_6H_4$ | 2-$CF_3$-$C_6H_4$ | N | |
| 128 | 4-$CH_3$-$C_6H_4$ | 2-F-$C_6H_4$ | N | |
| 129 | 4-$CH_3$-$C_6H_4$ | 2-Cl-$C_6H_4$ | N | |
| 130 | 4-$CH_3$-$C_6H_4$ | 2-Br-$C_6H_4$ | N | |
| 131 | 4-$CH_3$-$C_6H_4$ | 2-$CH_3$-$C_6H_4$ | N | |
| 132 | 4-$CH_3$-$C_6H_4$ | 2-$OCH_3$-$C_6H_4$ | N | |
| 133 | 4-$CH_3$-$C_6H_4$ | Cyclohexyl | N | |
| 134 | 2-$CH_3$-$C_6H_4$ | 2-F-$C_6H_4$ | N | |
| 135 | 2-$CH_3$-$C_6H_4$ | 2-Cl-$C_6H_4$ | N | |
| 136 | 2-$CH_3$-$C_6H_4$ | 2-$OCH_3$-$C_6H_4$ | N | |
| 137 | 2-$OCH_3$-$C_6H_4$ | 2-F-$C_6H_4$ | N | |
| 138 | 2-$OCH_3$-$C_6H_4$ | 2-Cl-$C_6H_4$ | N | |

Tabelle (Fortsetzung)

| Beispiel | A | B | X | Schmp/IR |
|---|---|---|---|---|
| 139 | 2-$OCH_3$-$C_6H_4$ | 2-$CH_3$-$C_6H_4$ | N | |
| 140 | 4-$OCH_3$-$C_6H_4$ | 2-$CF_3$-$C_6H_4$ | N | |
| 141 | 4-$OCH_3$-$C_6H_4$ | 2-F-$C_6H_4$ | N | |
| 142 | 4-$OCH_3$-$C_6H_4$ | 2-Cl-$C_6H_4$ | N | |
| 143 | 4-$OCH_3$-$C_6H_4$ | 2-Br-$C_6H_4$ | N | |
| 144 | 4-$OCH_3$-$C_6H_4$ | 2-$CH_3$-$C_6H_4$ | N | |
| 145 | 4-$NO_2$-$C_6H_4$ | 2-F-$C_6H_4$ | N | |
| 146 | 4-$NO_2$-$C_6H_4$ | 2-Cl-$C_6H_4$ | N | |
| 147 | 4-$NO_2$-$C_6H_4$ | 2-$CH_3$-$C_6H_4$ | N | |
| 148 | 4-$NH_2$-$C_6H_4$ | 2-F-$C_6H_4$ | N | |
| 149 | 4-$NH_2$-$C_6H_4$ | 2-Cl-$C_6H_4$ | N | |
| 150 | 4-$C_6H_5O$-$C_6H_4$ | 2-F-$C_6H_4$ | N | |
| 151 | 4-$C_6H_5O$-$C_6H_4$ | 2-Cl-$C_6H_4$ | N | |
| 152 | 4-$C_6H_5O$-$C_6H_4$ | 2-Br-$C_6H_4$ | N | |
| 153 | 4-$C_6H_5O$-$C_6H_4$ | 2-$CH_3$-$C_6H_4$ | N | |
| 154 | 4-$C_6H_5O$-$C_6H_4$ | 2-$CF_3$-$C_6H_4$ | N | |
| 155 | 4-$C_6H_5O$-$C_6H_4$ | 2-$OCH_3$-$C_6H_4$ | N | |
| 156 | 4-$C_6H_5O$-$C_6H_4$ | Cyclohexyl | N | |
| 157 | $CH_3$ | $C_6H_5$ | N | |
| 158 | $CH_3$ | 2-F-$C_6H_4$ | N | |
| 159 | $CH_3$ | 3-F-$C_6H_4$ | N | |
| 160 | $CH_3$ | 4-F-$C_6H_4$ | N | |
| 161 | $CH_3$ | 2-Cl-$C_6H_4$ | N | |
| 162 | $CH_3$ | 3-Cl-$C_6H_4$ | N | |

Tabelle (Fortsetzung)

| Beispiel | A | B | X | Schmp/IR |
|---|---|---|---|---|
| 163 | $CH_3$ | 4-Cl-$C_6H_4$ | N | |
| 164 | $CH_3$ | 2-Br-$C_6H_4$ | N | |
| 165 | $CH_3$ | 4-Br-$C_6H_4$ | N | |
| 166 | $CH_3$ | 2-$CH_3$-$C_6H_4$ | N | |
| 167 | $CH_3$ | 4-$CH_3$-$C_6H_4$ | N | |
| 168 | $CH_3$ | 2,4-$(CH_3)_2$-$C_6H_3$ | N | |
| 169 | $CH_3$ | 4-tert.-$C_4H_9$-$C_6H_4$ | N | |
| 170 | $CH_3$ | 2-$OCH_3$-$C_6H_4$ | N | |
| 171 | $CH_3$ | 1-Naphthyl | N | |
| 172 | $CH_3$ | 2-Naphthyl | N | |
| 173 | $CH_3$ | 4-Biphenyl | N | |
| 174 | $CH_3$ | 2,4-$Cl_2$-$C_6H_3$ | N | |
| 175 | $CH_3$ | 2-Cl-4-F-$C_6H_3$ | N | |
| 176 | $CH_3$ | 3-$NO_2$-$C_6H_4$ | N | |
| 177 | $CH_3$ | 4-$NH_2$-$C_6H_4$ | N | |
| 178 | $CH_3$ | Cyclohexyl | N | |
| 179 | tert.-$C_4H_9$ | $C_6H_5$ | N | |
| 180 | tert.-$C_4H_9$ | 2-F-$C_6H_4$ | N | |
| 181 | tert.-$C_4H_9$ | 2-Cl-$C_6H_4$ | N | |
| 182 | tert.-$C_4H_9$ | 2-Br-$C_6H_4$ | N | |
| 183 | tert.-$C_4H_9$ | 3-F-$C_6H_4$ | N | |
| 184 | tert.-$C_4H_9$ | 3-Cl-$C_6H_4$ | N | |
| 185 | tert.-$C_4H_9$ | 4-F-$C_6H_4$ | N | |
| 186 | tert.-$C_4H_9$ | 4-Cl-$C_6H_3$ | N | 2963, 1505, 1488, 1275, 1014, 844 $cm^{-1}$ |

Tabelle (Fortsetzung)

| Beispiel | A | B | X | Schmp/IR |
|---|---|---|---|---|
| 187 | tert.-$C_4H_9$ | 2-$CH_3$-$C_6H_4$ | N | |
| 188 | tert.-$C_4H_9$ | 4-$CH_3$-$C_6H_4$ | N | |
| 189 | tert.-$C_4H_9$ | 2,4-$(CH_3)_2$-$C_6H_3$ | N | |
| 190 | tert.-$C_6H_9$ | 4-tert.-$C_4H_9$-$C_6H_4$ | N | |
| 191 | tert.-$C_4H_9$ | 2-$OCH_3$-$C_6H_4$ | N | |
| 192 | tert.-$C_4H_9$ | 2,4-$Cl_2$-$C_6H_3$ | N | |
| 193 | tert.-$C_4H_9$ | 2-Naphthyl | N | |
| 194 | tert.-$C_4H_9$ | Cyclopentyl | N | |
| 195 | tert.-$C_4H_9$ | Cyclohexyl | N | |
| 196 | Cyclohexyl | $C_6H_5$ | N | |
| 197 | Cyclohexyl | 2-F-$C_6H_4$ | N | |
| 198 | Cyclohexyl | 3-F-$C_6H_4$ | N | |
| 199 | Cyclohexyl | 4-F-$C_6H_4$ | N | |
| 200 | Cyclohexyl | 2-Cl-$C_6H_4$ | N | |
| 201 | Cyclohexyl | 3-Cl-$C_6H_4$ | N | |
| 202 | Cyclohexyl | 4-Cl-$C_6H_4$ | N | 94°C |
| 203 | Cyclohexyl | 2-Br-$C_6H_4$ | N | |
| 204 | Cyclohexyl | 2-$CH_3$-$C_6H_4$ | N | |
| 205 | Cyclohexyl | 4-$CH_3$-$C_6H_4$ | N | |
| 206 | Cyclohexyl | 2,4$(CH_3)_2$-$C_6H_3$ | N | |
| 207 | Cyclohexyl | 2-$CF_3$-$C_6H_4$ | N | |
| 208 | Cyclohexyl | 3-$CF_3$-$C_6H_4$ | N | |
| 209 | Cyclohexyl | 4-$CF_3$-$C_6H_4$ | N | |
| 210 | Cyclohexyl | 4-tert.-$C_4H_9$-$C_6H_4$ | N | |

Tabelle (Fortsetzung)

| Beispiel | A | B | X | Schmp/IR |
|---|---|---|---|---|
| 211 | Cyclohexyl | 2-$OCH_3$-$C_6H_4$ | N | 112-115°C |
| 212 | Cyclohexyl | 2,4-$Cl_2$-$C_6H_3$ | N | |
| 213 | Cyclohexyl | 2-Naphthyl | N | |
| 214 | Cyclohexyl | 4-Biphenyl | N | |
| 215 | Cyclohexyl | 2-Cl-4-F-$C_6H_3$ | N | |
| 216 | Cyclohexyl | Cyclohexyl | N | |
| 217 | 4-Tetrahydropyranyl | $C_6H_5$ | N | |
| 218 | 4-Tetrahydropyranyl | 2-F-$C_6H_4$ | N | |
| 219 | 4-Tetrahydropyranyl | 3-F-$C_6H_4$ | N | |
| 220 | 4-Tetrahydropyranyl | 4-F-$C_6H_4$ | N | |
| 221 | 4-Tetrahydropyranyl | 2-Cl-$C_6H_4$ | N | |
| 222 | 4-Tetrahydropyranyl | 3-Cl-$C_6H_4$ | N | 164-166°C |
| 223 | 4-Tetrahydropyranyl | 4-Cl-$C_6H_4$ | N | |
| 224 | 4-Tetrahydropyranyl | 2-Br-$C_6H_4$ | N | |
| 225 | 4-Tetrahydropyranyl | 4-Br-$C_6H_4$ | N | |
| 226 | 4-Tetrahydropyranyl | 2-$CH_3$-$C_6H_4$ | N | |
| 227 | 4-Tetrahydropyranyl | 4-$CH_3$-$C_6H_4$ | N | |
| 228 | 4-Tetrahydropyranyl | 2,4-$(CH_3)_2$-$C_6H_3$ | N | |
| 229 | 4-Tetrahydropyranyl | 2-$OCH_3$-$C_6H_4$ | N | |
| 230 | 4-Tetrahydropyranyl | 2,4-$Cl_2$-$C_6H_3$ | N | |
| 231 | 4-Tetrahydropyranyl | 2-Naphthyl | N | |
| 232 | 4-Tetrahydropyranyl | 4-Biphenyl | N | |
| 233 | 4-Tetrahydropyranyl | Cyclohexyl | N | |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,

Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora Infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze. Es wurden also die Pilze oder die vor Pilzbefall zu schützenden Pflanzen, Saatgüter, Materialien oder der Boden mit einer fungizid wirksamen Menge des Wirkstoffs behandelt.

Die neuen Verbindungen können praktisch alle Entwicklungstadien einer Pflanze verschiedenartig beeinflussen und werden deshalb als Wachstums regulatoren eingesetzt. Die Wirkungsvielfalt der Pflanzenwachstumsregulatoren hängt ab vor allem

a) von der Pflanzenart und -sorte,
b) von dem Zeitpunkt der Applikation, bezogen auf das Entwicklungsstadium der Pflanze und von der Jahreszeit,
c) von dem Applikationsort und -verfahren (Samenbeize, Bodenbehandlung oder Blattapplikation)
d) von klimatischen Faktoren, z.B. Temperatur, Niederschlagsmenge, außerdem auch Tageslänge und Lichtintensität
e) von der Bodenbeschaffenheit (einschließlich Düngung),
f) von der Formulierung bzw. Anwendungsform des Wirkstoffs und schließlich
g) von den angewendeten Konzentrationen der aktiven Substanz.

Aus der Reihe der verschiedenartigen Anwendungsmöglichkeiten der erfindungsgemäßen Pflanzenwachstumsregulatoren im Pflanzenanbau, werden einige nachstehend erwähnt.

A.

Mit den erfindungsgemäß verwendbaren Verbindungen läßt sich das vegetative Wachstum der Pflanzen stark hemmen, was sich insbesondere in einer Reduzierung des Längenwachstums äußert. Die behandelten Pflanzen weisen demgemäß einen gedrungenen Wuchs auf; außerdem ist eine dunklere Blattfärbung zu beobachten.

Als vorteilhaft für die Praxis erweist sich z.B. die Verringerung des Grasbewuchses an Straßenrändern, Hecken, Kanalböschungen und auf Rasenflächen wie Park-, Sport- und Obstanlagen, Zierrasen und Flugplätzen, so daß der arbeits- und kostenaufwendige Rasenschnitt reduziert werden kann.

Von wirtschaftlichem Interesse ist auch die Erhöhung der Standfestigkeit von lageranfälligen Kulturen wie Getreide, Mais, Reis, Sonnenblumen und Soja. Die dabei verursachte Halmverkürzung und Halmverstärkung verringern oder beseitigen die Gefahr des "Lagerns" (des Umknickens) von Pflanzen unter ungünstigen Witterungsbedingungen vor der Ernte.

Wichtig ist auch die Anwendung von Wachstumsregulatoren zur Hemmung des Längenwachstums und zur zeitlichen Veränderung des Reifeverlaufs bei Baumwolle. Damit wird ein vollständig mechanisiertes Beernten dieser wichtigen Kulturpflanze ermöglicht.

Durch Anwendung von Wachstumsregulatoren kann auch die seitliche Verzweigung der Pflanzen vermehrt oder gehemmt werden. Daran besteht Interesse, wenn z.B. bei Tabakpflanzen die Ausbildung von Seitentrieben (Geiztrieben) zugunsten des Blattwachstums gehemmt werden soll.

Mit Wachstumsregulatoren läßt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt.

Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum Übergang in die generativen Phase neigen. Auch bei anderen Kulturen, z.B. Wintergetreide ist es vorteilhaft, wenn die Bestände durch Behandlung mit erfindungsgemäßen Verbindungen im Herbst zwar gut bestockt werden, aber nicht zu üppig in den Winter hineingehen. Dadurch kann der erhöhten Frostempfindlichkeit und - wegen der relativ geringen Blatt- und Pflanzenmasse - dem Befall mit verschiedenen Krankheiten (z.B. Pilzkrankheit) vorgebeugt werden. Die Hemmung des vegetativen Wachstums ermöglicht außerdem bei vielen Kulturpflanzen die dichtere Bepflanzung des Bodens, so daß ein Mehrertrag, bezogen auf die Bodenfläche, erzielt werden kann.

B.

Mit den neuen Mitteln lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchten zu erhöhen, den Proteingehalt in Getreide oder Soja zu steigern oder Gummibäume zum vermehrten Latexfluß zu stimulieren.

Dabei können die Verbindungen der Formel I Ertragssteigerungen durch Eingriffe in den pflanzlichen Stoffwechsel bzw. durch Förderung oder Hemmung des vegetativen und/oder des generativen Wachstums verursachen.

C.

Mit Pflanzenwachstumsregulatoren lassen sich schließlich sowohl eine Verkürzung bzw. Verlängerung der Entwicklungsstadien als auch eine Beschleunigung bzw. Verzögerung der Reife der geernteten Pflanzenteile vor oder nach der Ernte erreichen.

Von wirtschaftichem Interesse ist beispielsweise die Ernteerleicherung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heißt die Förderung der Ausbildung von Trenngewebe zwischen Frucht- bzw. Blatt- und Sproßteil der Pflanze ist auch für ein gut kontrollierbares Entblättern von Nutzpflanzen wesentlich.

D.

Mit Wachstumsregulatoren kann weiterhin der Wasserverbrauch von Pflanzen reduziert werden. Dies ist besonders wichtig für landwirtschaftliche Nutzflächen, die unter einem hohen Kostenaufwand künstlich bewässert werden müssen, z.B. in ariden oder semiariden Gebieten. Durch den Einsatz der erfindungsgemäßen Substanzen läßt sich die Intensität der Bewässerung reduzieren und damit eine kostengünstigere Bewirtschaftung durchführen. Unter dem Einfluß von Wachstumsregulatoren kommt es zu einer besseren Ausnutzung des vorhandenen Wassers, weil u.a.

- die Öffnungsweite des Stomata reduziert wird
- eine dickere Epidermis und Cuticula ausgebildet werden
- die Durchwurzelung des Bodens verbessert wird
- das Mikroklima im Pflanzenbestand durch einen kompakteren Wuchs günstig beeinflußt wird.

Die erfindungsgemäß zu verwendeten Wirkstoffe können den Kulturpflanzen sowohl vom Samen her (als Saatgutbeizmittel) als auch über den Boden, d.h. durch die Wurzel sowie über das Blatt zugeführt werden.

Infolge der hohen Pflanzenverträglichkeit kann die Aufwandmenge als Wachstumsregulatoren stark variiert werden.

Bei der Saatgutbehandlung werden im allgemeinen Wirkstoffmengen von 0,001 bis 50 g, vorzugsweise 0,01 bis 10 g je Kilogramm Saatgut benötigt.

Für die Blatt- und Bodenbehandlung sind im allgemeinen Gaben von 0,01 bis kg/ha, bevorzugt 0,02 bis 3 kg/ha, als ausreichend zu betrachten.

Die neuen Substanzen können in die üblichen Formulierungen übergeführt werden, wie Lösungen,

Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. PolyoxyethylenFettalkohol-Ether, Alkylsulfonate und Arylsulfonate) und Dispergiermittel, wie Lignin-Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz eingesetzt werden, z.B. gegen Paecilomyces variotii.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweiise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 25 mit 10 Gew.Teilen N-Methyl-α-pyrrolidon und erhält eine Lösung, die zur Anwendung in Form kleinster Tropfen geeignet ist.

II. 20 Gew.-Teile der Verbindung Nr. 27 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 25 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 27 werden in einer Mischung gelöst, die aus 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 25 werden mit 3 Gew.-Teilen des Natriumsalzes der Diisobutylnaphthalin-α-sulfonsäure, 10 Gew.Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 27 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 25 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 27 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 25 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Anwendungsbeispiele

Als Vergleichswirkstoffe wurden 1-(1,2,4-Triazol-1-yl)-2-(4-chlorphenyl)-3-(2,4-dichlorphenyl)-1-propen-3-ol (A) und 1-(1,2,4-Triazol-1-yl)-2-(4-chlorphenyl)-3-phenyl-1-propen-3-ol (B) - bekannt aus EP 23 286 - benutzt.

Anwendungsbeispiel 1

Wirksamkeit gegen Pyrenophora teres

Gerstenkeimlinge der Sorte "Igri" wurden im Zweiblattstadium mit wäßrigen Suspensionen, die 80 % Wirkstoff und 20 % Emulgator in der Trockensubstanz enthielten, tropfnaß gespritzt. Nach 24 Stunden wurden die Pflanzen mit einer Sporensuspension des Pilzes Pyrenophora tres inokuliert und für 48 Stunden in eine Klimakammer mit hoher Luftfeuchtigkeit bei 18° C gestellt. Anschließend wurden die Pflanzen im Gewächshaus bei 20 bis 22° C und 70 % relativer Luftfeuchtigkeit für weitere 5 Tage kultiviert. Dann wurde das Ausmaß des Blattbefalls ermittelt.

Das Ergebnis zeigt, daß die Wirkstoffe 25 und 27 bei der Anwendung als 0,05 %ige (Gew.%) Spritzbrühe eine bessere fungizide Wirkung zeigen (90 %) als die bekannten Vergleichswirkstoffe A und B (30 %).

Anwendungsbeispiel 2

Zur Bestimmung der wachstumsregulierenden Eigenschaften der Prüfsubstanzen wurden Testpflanzen auf ausreichend mit Nährstoffen versorgtem Kultursubstrat in Kunststoffgefäßen von ca. 12,5 cm Durchmesser angezogen.

Im Vorauflaufverfahren wurden die Testsubstanzen in wäßriger Aufbereitung am Tage der Einsaat auf das Saatbett gegossen.

Im Nachauflaufverfahren wurden die zu prüfenden Substanzen in wäßriger Aufbereitung auf die Pflanzen gesprüht. Die beobachtete wachstumsregulierende Wirkung wurde bei Versuchsende durch Wuchshöhenmessung belegt. Die so gewonnenen Meßwerte wurden zur Wuchshöhe der unbehandelten Pflanzen in Relation gesetzt.

Gleichlaufend zur Reduzierung des Längenwachstums stieg die Farbintensität der Blätter an. Der erhöhte Chlorophyllgehalt läßt eine ebenfalls erhöhte Photosyntheserate und damit eine erhöhte Ertragsbildung erwarten.

Die Einzeldaten sind den folgenden Tabellen zu entnehmen.

Tabelle 1

| Sommerweizen "Ralle" | | |
|---|---|---|
| Nachauflauf-Blattbehandlung | | |
| Wirkstoff | mg Wirkstoff je Gefäß | Wuchshöhen relativ |
| unbehandelt | - | 100 |
| B | 6 | 100 |
| A | 6 | 100 |
| 186 | 6 | 83,4 |

Tabelle 2

| Sommergerste "Aramir" | | |
|---|---|---|
| Vorauflauf-Blattbehandlung | | |
| Wirkstoff | Konz. mg WS/Gef. | Wuchshöhen relativ |
| unbehandelt | - | 100 |
| B | 6 | 47,7* |
| A | 6 | 100 |
| 25 | 6 | 24,9 |
| 186 | 6 | 27,1 |
| 202 | 6 | 33,2 |
| 212 | 6 | 33,2 |

* = deutliche Blattschäden

Tabelle 3

| Sommerraps "Petranova" | | |
|---|---|---|
| Vorauflauf-Bodenbehandlung | | |
| Wirkstoff | Konz. mg WS/Gefäß | Wuchshöhen relativ |
| unbehandelt | - | 100 |
| B | 6 | 86,5* |
| A | 6 | 100 |
| 186 | 6 | 70,2 |

* = starke Blattschäden

Tabelle 4

| Sommerraps "Petranova" | | |
|---|---|---|
| Nachauflauf-Blattbehandlung | | |
| Wirkstoff | Konz. mg WS/Gef. | Wuchshöhen relativ |
| unbehandelt | - | 100 |
| B | 6 | 81,1* |
| A | 6 | 86,6 |
| 186 | 6 | 74,3 |

* = deutliche Blattschäden

**Ansprüche**

1. Vinylazole der allgemeinen Formel I

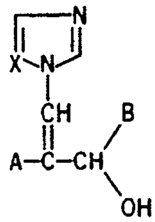

I

in welcher

A und B

gleich oder verschieden sind und $C_1$-$C_8$-Alkyl, $C_3$-$C_8$-Cycloalkyl, Tetrahydropyranyl, Pyridyl, Naphthyl, Biphenyl oder Phenyl bedeuten, wobei diese Reste einfach bis dreifach durch Halogen, Nitro, Phenoxy, Amino, Alkyl, Alkoxy oder Halogenalkyl mit jeweils 1 bis 4 C-Atomen substituiert sein können,

X

den Rest CH oder N bedeutet,

sowie deren für Pflanzen verträgliche Säureadditionssalze und Metallkomplexe außer den Verbindungen, in denen gleichzeitig A gegebenenfalls durch Chlor substituiertes Phenyl und B gegebenenfalls durch Chlor in 4-Stellung oder 2,4-Stellung substituiertes Phenyl bedeutet.

2. Verfahren zur Herstellung der Vinylazole der Formel I

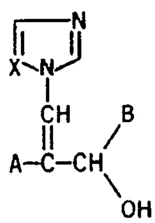

I

in welcher

A und B

gleich oder verschieden sind und $C_1$-$C_8$-Alkyl, $C_3$-$C_8$-Cycloalkyl, Tetrahydropyranyl, Pyridyl, Naphthyl, Biphenyl oder Phenyl bedeuten, wobei diese Reste einfach bis dreifach durch Halogen, Nitro, Phenoxy, Amino, Alkyl, Alkoxy oder Halogenalkyl mit jeweils 1 bis 4 C-Atomen substituiert sein können,

X

den Rest CH oder N bedeutet,

sowie deren für Pflanzen verträgliche Säureadditionssalze und Metallkomplexe auper den Verbindungen, in denen gleichzeitig A gegebenenfalls durch Chlor substituiertes Phenyl und B gegebenenfalls durch Chlor in 4-Stellung oder 2,4-Stellung substituiertes Phenyl bedeutet, dadurch gekennzeichnet, daß man eine Verbindung der Formel II,

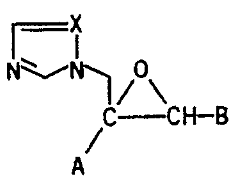

II

in welcher A, B und X die oben angegebene Bedeutung haben, in Gegenwart einer Base in die Vinylazole umlagert und die so erhaltenen Verbindungen gegebenenfalls in ihre für Pflanzen verträglichen Säureadditionssalze oder Metallkomplexe überführt.

3. Fungizides Mittel, enthaltend einen Trägerstoff und eine fungizid wirksame Menge eines Vinylazols der allgemeinen Formel I,

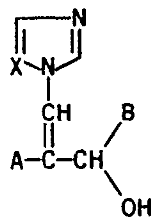

I

in welcher
A und B
gleich oder verschieden sind und $C_1$-$C_8$-Alkyl, $C_3$-$C_8$-Cycloalkyl, Tetrahydropyranyl, Pyridyl, Naphthyl, Biphenyl oder Phenyl bedeuten, wobei diese Reste einfach bis dreifach durch Halogen, Nitro, Phenoxy, Amino, Alkyl, Alkoxy oder Halogenalkyl mit jeweils 1 bis 4 C-Atomen substituiert sein können,
X
den Rest CH oder N bedeutet,
oder dessen für Pflanzen verträgliches Säureadditionssalz oder Metallkomplex außer den Verbindungen, in denen gleichzeitig A gegebenenfalls durch Chlor substituiertes Phenyl und B gegebenenfalls durch Chlor in 4-Stellung oder 2,4-Stellung substituiertes Phenyl bedeutet.

4. Verfahren zur Bekämpfung von Pilzen, dadurch gekennzeichnet, daß man eine fungizid wirksame Menge eine Vinylazols der Formel I,

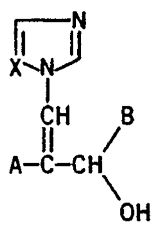

I

in welcher
A und B
gleich oder verschieden sind und $C_1$-$C_8$-Alkyl, $C_3$-$C_8$-Cycloalkyl, Tetrahydropyranyl, Pyridyl, Naphthyl, Biphenyl oder Phenyl bedeuten, wobei diese Reste einfach bis dreifach durch Halogen, Nitro, Phenoxy, Amino, Alkyl, Alkoxy oder Halogenalkyl mit jeweils 1 bis 4 C-Atomen substituiert sein können,
X
den Rest CH oder N bedeutet,
oder dessen für Pflanzen verträgliche Säureadditionssalze oder Metallkomplexe außer den Verbindungen, in denen gleichzeitig A gegebenenfalls durch Chlor substituiertes Phenyl und B gegebenenfalls durch Chlor in 4-Stellung oder 2,4-Stellung substituiertes Phenyl bedeutet auf die Pilze oder durch Pilzbefall bedrohte Materialien, Flächen, Pflanzen oder Saatgüter einwirken läßt.

5. Wachstumsregulierendes Mittel, enthaltend einen Trägerstoff und eine wachstumsregulierend wirksame Menge eines Vinylazols der Formel I,

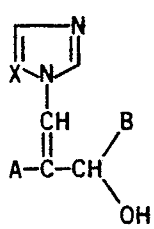

I

in welcher
A und B
gleich oder verschieden sind und $C_1$-$C_8$-Alkyl, $C_3$-$C_8$-Cycloalkyl, Tetrahydropyranyl, Pyridyl, Naphthyl, Biphenyl oder Phenyl bedeuten, wobei diese Reste einfach bis dreifach durch Halogen, Nitro, Phenoxy, Amino, Alkyl, Alkoxy oder Halogenalkyl mit jeweils 1 bis 4 C-Atomen substituiert sein können,

X
den Rest CH oder N bedeutet,
oder dessen für Pflanzen verträgliches Säureadditionssalz oder Metallkomplex auper den Verbindungen, in denen gleichzeitig A gegebenenfalls durch Chlor substituiertes Phenyl und B gegebenenfalls durch Chlor in 4-Stellung oder 2,4-Stellung substituiertes Phenyl bedeutet.

6. Verfahren zur Regulierung des Pflanzenwachstums, dadurch gekennzeichnet, daß man eine wachstumsregulierend wirksame Menge eines Vinylazols der Formel I,

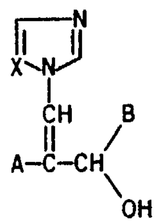

I

in welcher
A und B
gleich oder verschieden sind und $C_1$-$C_8$-Alkyl, $C_3$-$C_8$-Cycloalkyl, Tetrahydropyranyl, Pyridyl, Naphthyl, Biphenyl oder Phenyl bedeuten, wobei diese Reste einfach bis dreifach durch Halogen, Nitro, Phenoxy, Amino, Alkyl, Alkoxy oder Halogenalkyl mit jeweils 1 bis 4 C-Atomen substituiert sein können,
X
den Rest CH oder N bedeutet,
oder dessen für Pflanzen verträgliches Säureadditionssalz oder Metallkomplex außer den Verbindungen, in denen gleichzeitig A gegebenenfalls durch Chlor substituiertes Phenyl und B gegebenenfalls durch Chlor in 4-Stellung oder 2,4-Stellung substituiertes Phenyl bedeutet auf Kulturpflanzen oder den Boden einwirken läßt.

7. Vinylazol der Formel I gemäß Anspruch 1, dadurch gekennzeichnet, daß A 4-Fluorphenyl, B 2-Chlorphenyl und X N bedeutet.

8. Vinylazol der Formel I gemäß Anspruch 1, dadurch gekennzeichnet, daß A 4-Fluorphenyl, B 2-Bromphenyl und X N bedeutet.

9. Vinylazol der Formel I gemäß Anspruch 1, dadurch gekennzeichnet, daß A 4-Fluorphenyl, B 2-Trifluormethylphenyl und X N bedeutet.